# EUROPEAN PATENT SPECIFICATION

(11) **EP 1 154 270 B1**
(45) Date of publication and mention of the grant of the patent: **21.01.2015**
(21) Application number: 01111098.8
(22) Date of filing: 09.05.2001
(51) Int. Cl.: G01N 30/24, G01N 33/72, G01N 1/38

(54) **Glycated hemoglobin analyzer**
Analysator zur Messung von glykolisiertem Hämoglobin
Analyseur d'hémoglobine glucosée

(30) Priority: 09.05.2000 JP 2000142263; 09.05.2000 JP 2000142264; 09.05.2000 JP 2000142265; 09.05.2000 JP 2000142266
(43) Date of publication of application: 14.11.2001
(73) Proprietor: TOSOH CORPORATION, Shunan-shi, Yamaguchi-ken, 746-8501 (JP)
(72) Inventor: Yamagishi, Shigeo, Machida-shi, Tokyo (JP); Yoshikawa, Kazuhide, Machida-shi, Tokyo (JP); Matsuno, Takanori, Shunan-shi, Yamaguchi (JP)
(74) Representative: Pautex Schneider, Nicole Véronique

(56) References cited:
- EP-A- 0 299 419
- EP-A- 0 577 033
- EP-A- 0 800 073
- EP-A- 0 973 039
- WO-A-01/23879
- US-A- 4 954 253
- PATENT ABSTRACTS OF JAPAN vol. 1998, no. 02, 30 January 1998 (1998-01-30) & JP 09 274029 A (TOSOH CORP), 21 October 1997 (1997-10-21)

## Description

### BACKGROUND OF THE INVENTION

### 1. Field of the Invention

The present invention relates to a glycated hemoglobin analyzer using a liquid chromatography.

### 2. Description of the Related Art

A conventional glycated hemoglobin analyzer includes a separation column for separating a hemoglobin component, a detector for detecting a separated component, a liquid supplying pump for supplying an eluent, a sampling part for supplying a sample to be analyzed to an analyzer, and the like. In a clinical inspection equipment such as a glycated hemoglobin analyzer, in order to prevent the operator from suffering from his virus infection due to blood as a sample, it is common practice that a blood specimen collection tube containing collected blood is directly set to the analyzer, and then the analyzer automatically picks up and analyzes the sample.

In the glycated hemoglobin analyzer, a subject to be analyzed is a hemoglobin in a red blood cell. The hemoglobin is contained at high concentration in blood. Therefore, it is impossible to directly subject it to the liquid chromatography analysis. Accordingly, upon analyzing it, it is necessary to dilute the blood sample with about 100 times in advance. For this reason, in the glycated hemoglobin analyzer, it is a common practice that a trace of blood of several micro liters is collected, it is automatically diluted in the analyzer, and then it is analyzed.

For dilution, a sampling part is used, which is structured by a syringe unit for sucking and discharging a sample and a wash and hemolysis solution and a flow passage change-over mechanism combined with a plurality of 2- or 3-way electromagnetic valves, both being controlled by a control means (CPU) of the analyzer.

Such a conventional analyzer is described for example in the Japanese Patent Application JP-A-09 274029.

In the sampling part having the flow passage change-over mechanism including the combination of the plural electromagnetic valves, the flow passages arrangement is complicated because of the combination of the plural electromagnetic valves. Further, the electromagnetic valve contains movable parts therein, it contains many locations to be maintained. When the plurality of electromagnetic valves are disposed between the syringe for sample suction and a sample container, the flow passage is more increased in its length. Accordingly, the dead volume within the flow passage is large. This fact makes it difficult to reduce an air capacity within the flow passage extending between the syringe and the sample liquid surface. As a result, the precious sample loss is great, and it is difficult to suck an exact amount of the sample.

Further, when the plural electromagnetic valves are located between the syringe and the sample liquid surface, suction resistance values of the electromagnetic valves are accumulated in calculation. Therefore, it is difficult to increase a suction velocity of the syringe to a predetermined velocity value or higher. In some cases, the inside of the flow passage is temporarily under a negative pressure, and air is generated therein, and thus, the suction accuracy is degraded.

To reduce the dead volume, the syringe and the electromagnetic valves may be connected by use of flow passages are small in inside diameter. However, in this case, the suction resistance by the flow passage is large, and thus the suction velocity is hindered, and the suction accuracy is degraded. The difficulty of increasing the suction velocity also hinders an attempt to increase the operation speed of the whole sampling mechanism with the intention of reducing the analyzing time per sample.

### SUMMARY OF THE INVENTION

Accordingly, an object of the present invention is to provide a glycated hemoglobin analyzer which is provided with a flow passage change-over mechanism which can form a flow passage by use of only a single flow passage change-over valve of the rotary type, without using an electromagnetic valve, whereby the analyzer is capable of operating, at high speeds, a pump for sucking and discharging a sample and a wash and hemolysis solution at each step of sampling, and a time taken for a pretreatment for each sample is remarkably reduced.

The inventor made much efforts to achieve the above object, and successfully reached a technical idea of the invention. To achieve the above object, there is provided a glycated hemoglobin analyzer using a liquid chromatography, having a separation column for separating a hemoglobin component, a detector for detecting a separated component, a liquid supplying pump for supplying an eluent, and a sampling part for supplying a sample to be analyzed to an analyzing part, characterized in that the sampling part being constructed with a flow passage change-over mechanism constructed such that a flow passage is formed by use of a flow passage change-over valve of the rotary type. This flow passage change-over mechanism is a non-electromagnetic-type valve, not using an electromagnetic valve.

Here, the rotary type flow passage change-over valve used in the flow passage change-over mechanism of the invention may be any valve flow passage change-over valve, which utilizes a rotary valve, and includes more than three ports capable of connecting flow passages, and operates such that with rotation of the valve, a flow passage connection mechanism, called a rotary seal, interlockingly rotates to interconnect flow passages connecting to the valve and disconnect them from each other. The number of flow passages that may be connected to the valve is at least three. In a case where the flow passage change-over mechanism is assembled into the sampling mechanism to form flow passages for operations including operations of diluting the sample, cleaning the sampling needle and the like, more than six flow passages may be connected to the valve. In this case, it is preferable that one flow passage is connected to one of the remaining five flow passages or more (this one flow passage will frequently be denoted as (A)).

The flow passage change-over mechanism of the invention may be rearranged into a sampling mechanism for sucking and discharging samples and a wash and hemolysis solution when it is connected to drive means for sampling, such as a cylinder pump or a peristaltic pump, which is used by a general liquid chromatography apparatus. When the flow passage change-over mechanism, which is connected to the sampling drive means, is further connected to the sampling needle, a dilution liquid supply flow passage, a cleaning liquid supply flow passage, a dilution block, a waste liquid discharging flow passage and the like, it may be used as the auto sampler mentioned above. In order to simplify the structure of the whole sampling mechanism, it is effective to connect the sampling drive means to the flowpassage (A) . For the other means, it may be connected to an optimum port of the rotary valve in accordance with an operation sequence of the auto sampler. In the present invention, the rotary valve in the flow passage change-over mechanism may be driven in an desired manner.

In the invention, it may be driven manually or automatically by using the motor. In a case where it is assembled into the auto sampler for the liquid chromatography, it is preferable that it is automatically driven by using the motor.

### BRIEF DESCRIPTION OF THE DRAWINGS

Fig. 1 is a diagram schematically showing a flow passage arrangement for a glycated hemoglobin analyzer according to the present invention;
Fig. 2 is a front view showing a rotary valve;
Fig. 3 is a side view showing a portion of a main body of the rotary valve and a pulse motor connected to the rotary valve;
Fig. 4 is a sectional view showing an inner structure of the rotary valve;
Fig. 5 is a cross sectional view showing a plunger pump;
Fig. 6 is an enlarged view showing a portion indicated as VI in Fig. 5;
Fig. 7 is a diagram showing an arrangement of a cleaning mechanism in the plunger pump;
Fig. 8 is a perspective view showing a rack;
Fig. 9 is a diagram showing a state of identifying the sample container by a sample container identifying device;
Fig. 10 is another diagram showing a state of identifying the sample container by a sample container identifying device; and
Fig. 11 is still another diagram showing a state of identifying the sample container by a sample container identifying device.

### DETAILED DESCRIPTION OF THE PREFERRED EMBODIMENTS

A glycated hemoglobin analyzer based on the liquid chromatography which is an embodiment of the present invention will be described in detail with reference to the accompanying drawings. It should be understood that the invention is not limited to the analyzer illustrated in the drawings.

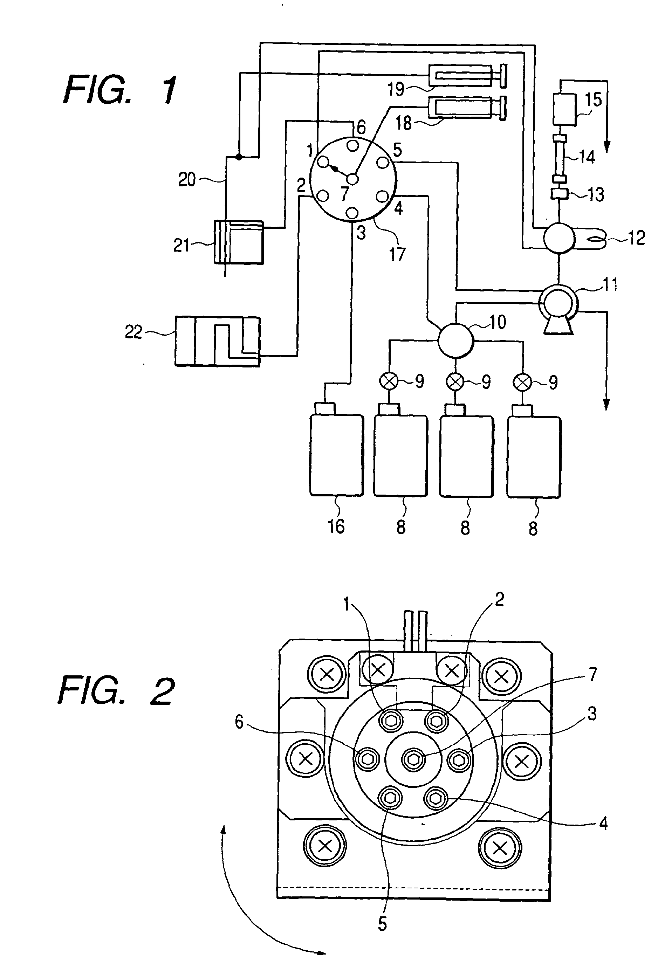

Fig. 1 is a diagram schematically showing a glycated hemoglobin analyzer constructed according to the present invention. The glycated hemoglobin analyzer includes an eluent 8, an eluent change-over electromagnetic valve 9, an eluent mixing block 10, an eluent supplying pump (a glycated hemoglobin analyzer pump) 11, a sample injection valve 12, a line filter 13, an analyzing column 14, a detector 15, a wash and hemolysis solution 16, a flow passage switch-over valve (a rotary valve) 17, a syringe pump 18, a micro-syringe pump 19, a sampling needle 20, a needle cleaning block 21, and a dilution block 22. The flow-passage switchover valve 17 is connected to the syringe pump 18, the wash and hemolysis solution part 16, the needle cleaning block 21, the dilution block 22, the sampling needle 20, the eluent supplying pump 11, and the eluent mixing block 10, whereby a flow passage change-over mechanism is formed.

Glycohemoglobin is measured in amanner that a blood sample is diluted with about 100 times by a dilution liquid, and then is separated by an ion exchanging chromatography. An operation of a sampling mechanism in the measurement will be described in successive order.

### 1) Collection of a blood sample

The rotary valve 17 is rotated to connect a port 1 to a port 7. The sampling needle 20 is put into the blood sample, and the syringe pump 19 is driven, so that a fixed amount of blood is sucked into the sampling needle 20.

### 2) Cleaning of the outer wall of the sampling needle

The rotary valve 17 is rotated to connect a port 3 to the port 7. Firstly, the syringe pump 18 is driven to suck a fixed amount of a wash and hemolysis solution 16 into the syringe.

Then, the rotary valve 17 is rotated to connect a port 6 to the port 7. In turn, the wash and hemolysis solution that has been sucked into the syringe flows to the needle cleaning block 21, so as to wash away the excessive blood sample attached to the outer wall of the needle.

### 3) Discharging of the blood sample to the dilution block and its mixing

The rotary valve 17 is rotated to connect the ports 3 and 7. Then, as in the previous case, the wash and hemolysis solution 16 is sucked into the syringe. Then, the rotary valve 17 is rotated to connect the ports 1 and 7, so that the blood sample of the needle, together with the wash and hemolysis solution 16 that has been sucked into the syringe, is discharged into the dilution block 22. Further, the sucking/discharging operation by the syringe pump 18 is repeated two or three times. As a result, the wash and hemolysis solution 16 and the blood in the dilution block 22 are sucked and discharged to mix them uniformly.

### 4) Introduction to the analyzer

The diluted sample in the dilution block 22 is introduced into the analyzer through the sample injection valve 12. A fixed-amount introducing valve is formed with a sampling probe and an injection valve connected to the sampling probe.

### 5) Cleaning

After the diluted sample is introduced into the analyzer, the inside of the sampling needle 20 and the dilution block 22 are washed out using the wash and hemolysis solution. To start with, the rotary valve 17 is turned to connect the ports 3 and 7. Then, the syringe pump 18 is driven to suck the wash and hemolysis solution from the syringe. Thereafter, the rotary valve 17 is driven to connect the ports 1 and 7, whereby the wash and hemolysis solution in the syringe is discharged from the sampling needle 20. As a result, the inner wall of the sampling needle 20 is washed out by the wash and hemolysis solution, and filled with the same. Then, the rotary valve 17 is rotated again to connect the ports 2 and 7, so that the wash and hemolysis solution filling the syringe is discharged into the dilution block 22. The dilution block has an overflow structure. The wash and hemolysis solution containing the diluted sample are completely discharged, so that the dilution block 22 is filled with the wash and hemolysis solution.

Repeating a sequence of the steps 1) to 5) mentioned above successively, the successive sampling and the analysis of the glycohemoglobin are performed.

Fig. 2 is a diagram showing an overall rotary valve available for the present invention. This rotary valve includes seven ports for interconnecting flow passages. With rotation of a rotor within the valve in directions of arrows, the center port 7 is connected to one of the remaining six ports 1 to 6.

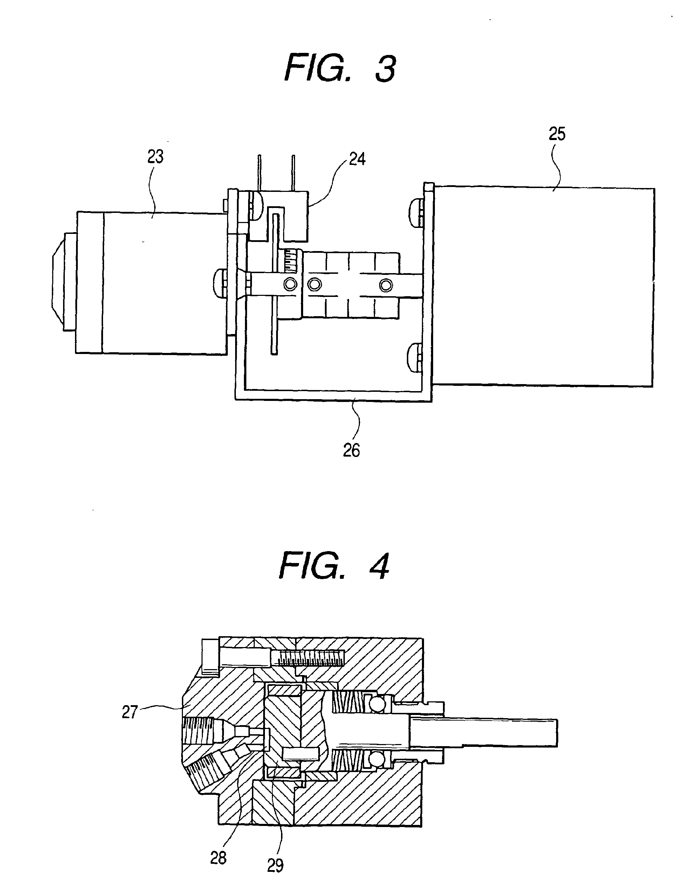

Fig. 3 is a side view showing a portion of a main body 23 of the rotary valve 17 of Fig. 2 and a pulse motor 25 connected to the main body 23 via a fixing member 26. The pulse motor 25 is connected along an elongation of the valve rotary shaft. The pulse motor 25 is controlled by an external control unit to turn the valve at a desired angle in conjunction with a rotating position detecting sensor 24, whereby the center port 7 is connected to a desired port of the remaining ones 1 to 6.

Fig. 4 is a sectional view showing an inner structure of the rotary valve 17. A rotor seal 29 having a groove 28 extended in only one direction is installed behind a stator 27 for connecting the flow passages. When the rotor seal 29 is rotated together with the shaft, the port to be connected to the center port is switched over to another. The rotary valve thus constructed is commercially available (from, for example, Rheodyne corporation in U.S.A.). It is evident that any other suitable valve having the inner structure different from that of the Fig. 4 may be used for the rotary valve of the invention.

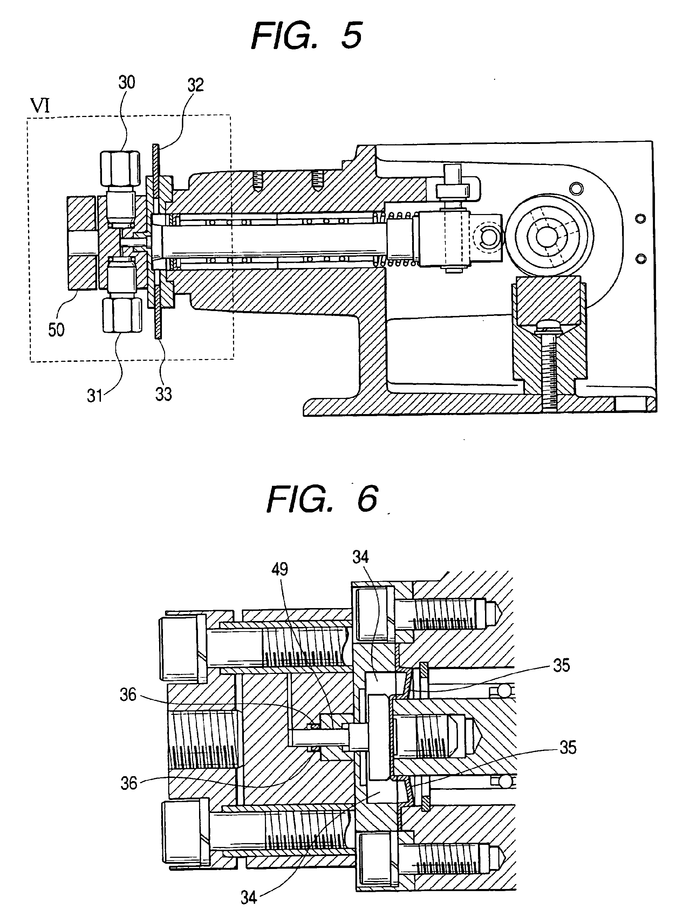

Figs. 5 and 6 show a example of a plunger pump adaptable for the glycated hemoglobin analyzer pump 11 according to the invention. In the plunger pump, a rotating motion of the motor is converted into a reciprocal motion of a plunger 49 by use of a mechanism including a cam and the like. In the reciprocating plunger 49, its tip is reciprocatively moved within a pump head 50, through a plunger seal 36. The inner volume of the pump head 50 increases and decreases in accordance with the reciprocative motion of the plunger 49. Check valves 30, 31 are provided at the inlet and the outlet of the pump head 50. With a volume variation, the liquid flows from the inlet to the outlet. In this way, the plunger pump feeds the liquid.

Since the plunger 49 passes through the pump head 50 via the seal, fine flaws are formed in the surfaces of the seal and the plunger 49, with use of the plunger pump. As a result, there is a possibility that a small amount of the liquid leaks to the back side of the pump head 50. Since the amount of the leaking liquid is very small, it does not hinder the operation of the plunger pump instantly. However, when it is used for a long time, salts contained in the liquid separate out onto the surfaces of the plunger and the seal. The separated salts rubs the plunger and seal surfaces, so that the lifetime of the plunger and the seal is considerably reduced.

In the present invention, it is preferable to wash out the plunger pump by using the cleaning liquid, which is used for washing the sampling part. In the invention, a liquid containing refined water and a small amount of surfactant (e.g., TritonX-100 of about 0.01%(V/V)) is used for the wash and homolysis solution 16. This solution is also used for the cleaning liquid for the plunger pump.

In the preferred embodiment of the invention, the solution is supplied through the sampling part. In this case, liquid feeding means, such as a cylinder pump in the sampling part is used for the liquid feeding. By the switching over in the sampling part, the solution flows to the flow passage led to the sampling part or the flow passage led to the plunger pump.

Fig. 5 is a cross sectional view showing the plunger pump. The liquid to be fed flows through a check valve 30 and flows out through a check valve 31. In the plunger pump used in the invention, cleaning liquid passage tubes 32 and 33 are further coupled thereto.

Fig. 6 is an enlarged view showing a portion indicated as A in Fig. 5. A portion in which the cleaning liquid flows is a space indicated by reference numeral 34. The space 34 is separated from a plunger operation mechanism on the right in the drawing by a diaphragm 35, and from the flow passage for the analysis by a plunger seal 36, thereby preventing the cleaning liquid from flowing out of the cleaning liquid passage tubes 32 and 33.

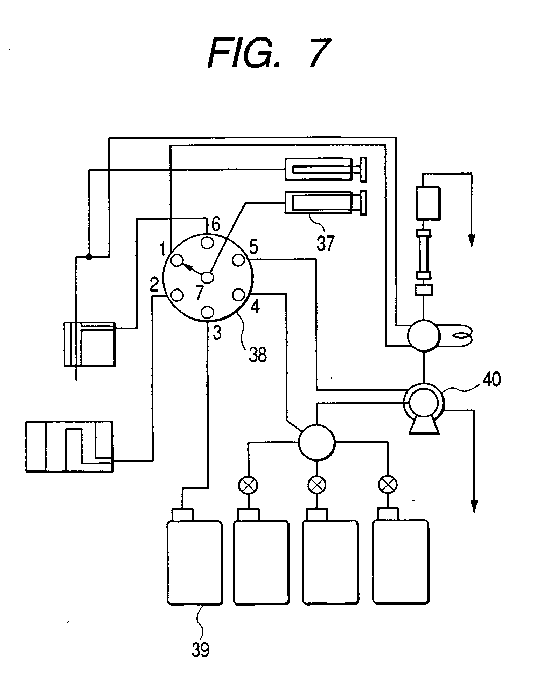

Fig. 7 is a diagram for explaining in detail the cleaning method mentioned above. The flow passage to be connected to the cylinder pump 37 is selected by a rotary valve 38. To wash the plunger of the pump, a cleaning liquid 39 is first sucked by the cylinder pump 37, then the flow passage is switched over to the pump washing line via the port 5, the sucked cleaning liquid passes through the pump washing line to wash out a pump 40, and then is discharged outside. Usually, it is satisfactory that such a cleaning operation is executed when the analyzer is started and ended. Accordingly, the cleaning operation is not performed when the analyzing operation is performed, and the washing line may be filled with the cleaning liquid.

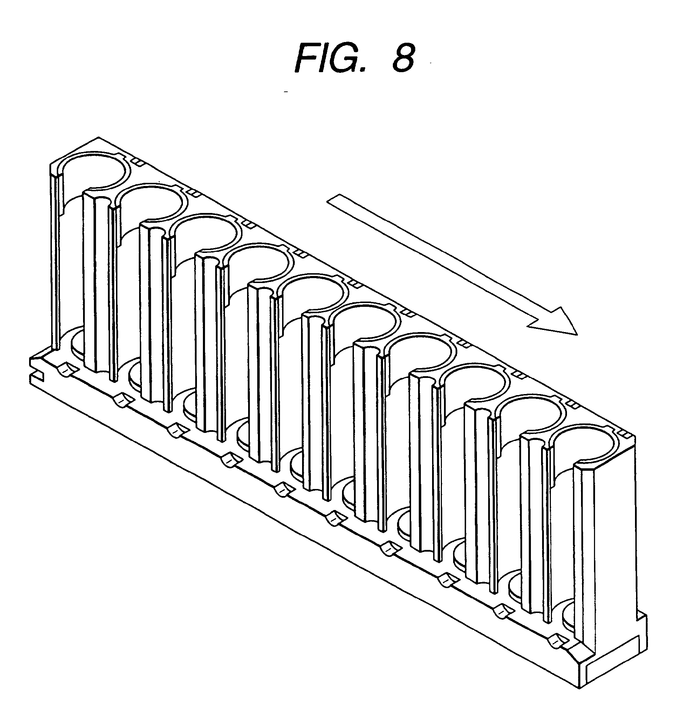

Although the analyzer of the invention handles the blood sample, in order to prevent the operator from being infected with virus and the like, it is preferable that a collected blood is not transferred from the blood specimen collection tube to a sample container specially prepared, and the blood specimen collection tube is utilized as the sample container as it is. Specifically, those tubes are set in a rack as shown in Fig. 8, and are continuously or intermittently transported to the sampling part of the invention.

In the analysis, a standard sample and a control sample are also used for the calibration of the analyzer and its accuracy management. Polypropylene containers of 1 to 2ml in volume, which are different from the sample collection tubes, are usually used for containing the standard sample or the control sample. In a case that a subject to be examined is an infant or the like, it is difficult to collect an amount of blood, which is equal to that collected from an adult. In this case, it is common that an injector, not the usual blood collection tube, is used for collecting blood, and the collected blood is put in the container of a small volume as mentioned above, and disposed on the rack.

In the analyzer, containers containing the blood sample are set in the rack, and are transported to the sampling part. Then, a fixed amount of blood sample necessary for the measurement is sucked in accordance with a predetermined operation sequence, and is subjected to the analysis. In this case, the blood collection tube and the polypropylene container of about 1 to 2ml are different in size and shape. If those tube and containers are transported while randomly arranged, the following problem arises. Those tubes and containers are different in the liquid level of the sample and the opening-to-bottom distance from each other. This fact hinders operation sequences of the descending control of the sample suction nozzle and the like.

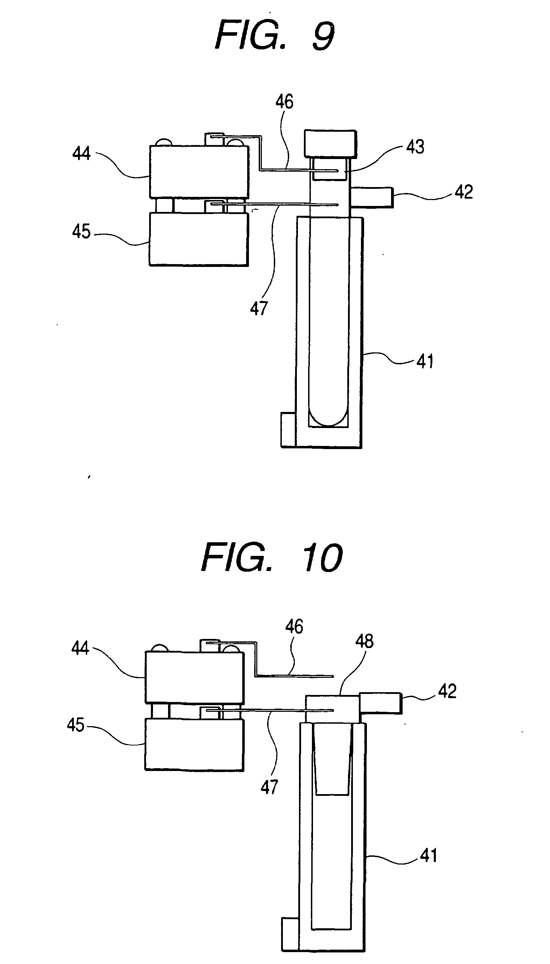

The present invention also provides an analyzer which is capable of automatically carrying out the analysis by equipping with a transporting means such a rack and a sample container identifying device as shown in Figs. 9 and 10. Figs. 9 and 10 show diagrams as viewed from this side when the sample container identifying device is viewed in the rack transporting direction. The sample container identifying device includes a rack 41, a container (blood collection tube) 43 disposed on the rack 41, and two actuator type micro-switches 44, 45. Those micro-switches 44, 45 are located at different mounting positions for discriminating a height difference of a part of the container, which is projected above the rack body. In this instance, the two micro-switches are vertically disposed one on the other at different height positions (viewed in the direction orthogonal to the container transporting direction), whereby the containers being transported can instantaneously be identified. Those micro-switches 44, 45 are in an off (on) state when the sample container is not set in the rack. When the container is transported and the actuator 46, 47 is inclined at about 30' or larger with respect to the horizontal direction, the micro-switches 44, 45 are turned on (off). When the rack is transported and the sample containers set on the rack reaches a position at which the micro-switches are mounted, if the transported sample container 43, the height of the part of which is projected above the rack body being higher than another sample container 48 is transported, the two micro-switches 44, 45 are both turned on (off). In addition, if the transported sample container 43, the height of the part of which is projected above the rack body being lower than another sample container 48 is transported, the lower micro-switch 45 of those micro-switches are both turned on (off), while the upper micro-switch 44 remains off (on).

In the illustrated case, the blood collection tube corresponds to the sample container 43, and a commercially available sample cup (used for the Hitachi automatic analyzer 703) corresponds to the sample container 48. Therefore, on the basis of the observation of the output signals from the two micro-switches, one detects whether the transported container is the sample container 43 or the sample container 48, or the container is set in the rack or not. Accordingly, the analyzer is controlled by the output signals so as to start a predetermined operation sequence. The micro-switches illustrated may be mounted on the lower side of the rack if the rack is so configured.

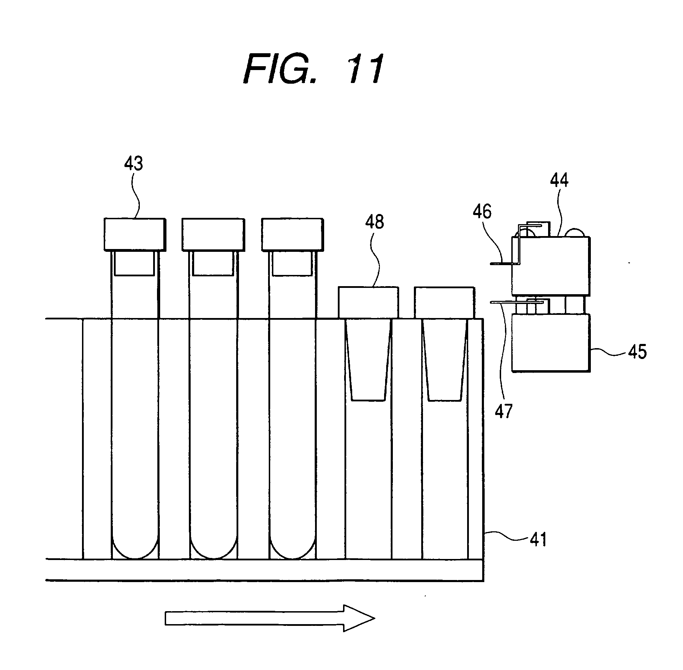

Fig. 11 shows how to identify the container shown in Figs. 9 and 10 when it is viewed from the rear side of the device (the sample container identifying device per is illustrated while being turned by 90°). In the figure, an arrow indicates an advancing direction of the rack 41. Although two kinds of containers 43 and 48 are set in the rack, with the movement of the rack, those containers are transported in the order of the sample container 48, the sample container 48, the sample container 43, the sample container 43, and the sample container 43. When the sample container 48 passes in the front of the micro-switches 44, 45, only a lower actuator 47 is operated. When the sample container 43 passes the front of the micro-switches 44, 45, the upper and lower actuators 46, 47 are both operated. Accordingly, the kinds of the containers set in the rack can readily be identified.

For the actuators, it is preferable to use the micro-switches which have actuators equal to or larger than the number of the kinds of containers to be identified. An n-number of kinds of containers can be identified by using an (n - 1) number of micro-switches. If an "n" number of micro-switches or larger are used, an "n" number of kinds of containers can be identified, and further whether or not the container is set in the rack can also be detected. Description will be given about a case that at least two micro-switches are used in a fixed state, while sample containers are transported. As an example, there are considered two cases. One is that the micro-switches are serially arranged in the same direction as the sample container transporting direction, and those containers are identified after all those containers pass in the front of the micro-switches. Alternatively, the other is that those micro-switches are arranged in the direction vertical to the container transporting direction, and the transporting containers are instantaneously identified. Generally, it is preferable that the latter arrangement of the micro-switches is used and the containers are instantaneously identified, in order to secure the size reduction of the sample container identifying device and the simplification of the operation sequence of the whole analyzer. The same thing is true for a case where the sample container identifying device (actuator type micro-switches) is moved, or a case where the sample container identifying device and the containers are moved relative to each other, as will be described later.

Regarding a specific arrangement of the actuator type micro-switches, a position at which one discriminates the containers in size and configuration is determined based on the kinds of the containers to be set in the rack, and any special limitation is not put in particular. For example, actuator type micro-switches are mounted at different height positions, and a height of the part of the container projected above the rack body and a diameter of the container are discriminately detected.

Actuator type micro-switches are not limited in particular if those are turned on and off depending on the size and/or configuration of the containers to be set (or to probably be set) in the rack or depending on whether or not the container is present. But, Of those actuator type micro-switches, the micro-switches of the lever type or the small torque rotation type are preferably used in order to secure the well adaptability and easy adjustment for various sizes and configurations of the containers. The micro-switches of the lever type may be a commercially available one (e.g., SS-01 type micro-switch, trade name, manufactured by Omron corporation). The micro-switches of the small torque rotation type may be a commercially available one (e.g., D2MC type micro-switch, trade name, manufactured by Omron corporation).

Any special limitation is not put on the shape of the rack and the number of containers that may be set in the rack. A rack being capable of receiving only one container or a plurality of containers may be used. As a specific example of such a rack, there is considered a rack capable of receiving one or a plurality of vacuum blood collection tube for collecting a blood sample. It is marketed as the Sysmex standard rack, manufactured by Sysmex corporation. This rack is capable of receiving one to ten blood collection tubes of 12 to 15 mm in diameter, and containers resembling them. The racks described above are all of the type in which the sample containers received are linearly arranged. If required, the rack of the type in which the containers received, like a turntable, are circularly arranged may be used.

The container to be identified by the sample container identifying means may be any suitable one if it is capable of holding the sample, in addition to the polypropylene container and the vacuum blood collection tube. An example of the container adaptable for the marketed rack is a vacuum blood collection tube of 75 to 100mm in height and 12 to 15mm in diameter. Such a vacuum blood collection container may commercially be available as VENOJECT II, trade name, manufactured by Terumo corporation. In addition, as an example other than the vacuum blood collection tube, a sample cup for Hitachi automatic analyzer 703, φ17 x 38mm, manufactured by UNIFLEX Co, Ltd. may also be enumerated.

For identification, the containers are moved so as to turn on or off the actuator type micro-switches installed to the sample container identifying device. Alternatively, the sample container identifying device (actuator type micro-switches) are moved so that those are turned on or off by the container. In another alternative, the sample containers and the sample container identifying device (actuator type micro-switches) are moved relative to each other so that the actuator type micro-switches are turned on and off by the containers. To simplify the construction of the whole analyzer, it is fairly preferable that the sample container identifying device of the invention is fixed, and the actuator type micro-switches of the sample container identifying device are turned on and off by the containers set in the rack through the movement of the rack. The movement of the rack and/or the sample container identifying device may be continuous or intermittent. Use of the intermittent movement is preferable to secure an easy control of the overall analyzer. An example of the control is to select appropriate operation sequences for different sample sucking nozzles, and to start the selected sequential operations.

As seen from the foregoing description, the invention employs the rotary valve in place of the combination of the electromagnetic valves. Accordingly, the glycated hemoglobin analyzer with the flow passage switch-over mechanism, which realizes a higher accurate sampling than the conventional one, is capable of operating, at high speeds, the pump for sucking and discharging the blood samples and the wash and hemolysis solution at the respective sampling stages, and remarkably reducing the time taken for the pretreatment per sample.

## Claims

1. A glycated hemoglobin analyzer using a liquid chromatography, comprising :
a sampling part (17, 18, 37, 38) for automatic dilution of a blood sample to create a diluted sample for analysis,
a sample injection valve (12) for supplying said diluted sample to be analyzed to a separation column;
a liquid supplying pump (11, 40) for supplying an eluent;
a separation column (14) for separating a hemoglobin component; and
a detector (15) for detecting the separated component;
**characterized in that** :
said sampling part (17, 18, 37, 38) comprises a flow passage change-over mechanism comprising a flow passage change-over valve of the rotary type (17, 38), and a syringe pump (18, 37),
wherein
said flow passage change-over valve is a non-electromagnetic valve type,
said flow passage change-over mechanism is constructed such that a flow passage is formed by a changeover of said flow passage change-over valve,
said flow passage change-over valve (17, 38) has at least three ports (1, 2, 3, 4, 5, 6, 7), and said flow passage change-over valve (17, 38) is changed over to connect two ports (1, 2, 3, 4, 5, 6, 7) to thereby form the flow passage,
said syringe pump (18, 37) is connected to the center port (7) of said flow passage change-over valve (17, 38),
a wash and hemolysis solution part (16) is connected to an other one port (1, 2, 3, 4, 5, 6) of said valve (17, 38) for sucking the wash and hemolysis solution (16) into said syringe pump (18, 37), and
said sample injection valve (12) is connected to an other one port ((1, 2, 3, 4, 5, 6) of said valve (17, 38)
(i) for discharging said blood sample sucked in a sampling needle (20) to a dilution block (22) for mixing and dilution,
(ii) for introducing a diluted sample to the sample injection valve (12) and
(iii) for cleaning the inside of the sampling needle (20).

2. The glycated hemoglobin analyzer according to claim 1, wherein said liquid supplying pump (11, 40) is a plunger pump including a pump head (50), a plunger (49) reciprocating within said pump head (50) through a plunger seal (36) and a partitioned chamber (34) separated from said pump head (50) by said plunger seal (36), and
the confronting surfaces of said plunger (49) and said plunger seal (36) are washed out by feeding a cleaning liquid to said partitioned chamber (34).

3. The glycated hemoglobin analyzer according to claim 2, wherein said liquid supplying pump (11, 40) is washed out by feeding the cleaning liquid by said syringe pump (18, 37) through the flow passage change-over valve (17, 38).

4. The glycated hemoglobin analyzer according to claim 1, further comprising :
a sample container identifying device for identifying containers (43, 48) set in a rack (41), said sample container identifying device including actuator type micro-switches (44, 45), the number of which is equal or more than the number of kinds of containers (43, 48) to be identified,
wherein the kinds of said containers (43, 48) are identified through the on and off states of said actuator type micro-switches (44, 45).

5. The glycated hemoglobin analyzer according to claim 4, wherein said sample container identifying device identifies the kinds of said containers (43, 48) during the intermittent movement of said containers (43, 48) set in the rack (41).

6. The glycated hemoglobin analyzer according to claim 4, wherein said sample container identifying device identifies the kinds of said containers on the basis of the height difference of said containers (43, 48) set in the rack (41).

7. The glycated hemoglobin analyzer according to claim 4, wherein the kinds of said containers to be identified includes a blood collection tube.

## Patentansprüche

1. Analysator zur Messung von glykolisiertem Hämoglobin durch Anwenden einer Flüssigkeitschromatographie, aufweisend:
ein Probenentnahmeteil (17, 18, 37, 38) zum automatischen Verdünnen einer Blutprobe zum Erzeugen einer verdünnten Probe für Analyse,
ein Probeneinspritzventil (12) für die Zufuhr der zu analysierenden verdünnten Probe zu einer Trennsäule;
eine Flüssigkeitszuführpumpe (11, 40) zum Zuführen eines Eluenten;
eine Trennsäule (14) zum Trennen einer Hämoglobinkomponente; und
einen Detektor (15) zum Detektieren der getrennten Komponente;
**dadurch gekennzeichnet, dass**:
das Probenentnahmeteil (17, 18, 37, 38) einen Flussdurchgang-Umschaltmechanismus, aufweisend ein Flussdurchgang-Umschaltventil vom Drehschiebertyp (17, 38) und eine Spritzenpumpe (18, 37), aufweist
wobei
das Flussdurchgang-Umschaltventil ein Ventil von einem nicht-elektromagnetischen Typ ist,
der Flussdurchgang-Umschaltmechanismus so konstruiert ist, dass ein Flussdurchgang durch ein Umschalten des Flussdurchgang-Umschaltventils gebildet wird,
das Flussdurchgang-Umschaltventil (17, 38) mindestens drei Anschlüsse (1, 2, 3, 4, 5, 6, 7) aufweist, und das Flussdurchgang-Umschaltventil (17, 38) umgeschaltet wird, um zwei Anschlüsse (1, 2, 3, 4, 5, 6, 7) miteinander zu verbinden, um dadurch den Flussdurchgang zu bilden,
die Spritzenpumpe (18, 37) an den mittleren Anschluss (7) des Flussdurchgang-Umschaltventils (17, 38) angeschlossen ist,
ein Wasch- und Hämolyselösungsteil (16) an einen anderen Anschluss (1, 2, 3, 4, 5, 6) des Ventils (17, 38) zum Saugen der Wasch- und Hämolyselösung (16) in die Spritzenpumpe (18, 37) angeschlossen ist, und
das Probeneinspritzventil (12) an einen anderen Anschluss (1, 2, 3, 4, 5, 6) des Ventils (17, 38)
(i) zum Ausgeben der in eine Probenentnahmenadel (20) eingesaugten Blutprobe in einen Verdünnungsblock (22) zum Mischen und Verdünnen,
(ii) zum Einführen einer verdünnten Probe in das Probeneinspritzventil (12) und
(iii) zum Reinigen der Innenseite der Probenentnahmenadel (20) angeschlossen ist.

2. Analysator zur Messung von glykolisiertem Hämoglobin nach Anspruch 1, wobei die Flüssigkeitszuführpumpe (11, 40) eine Plungerpumpe ist, aufweisend einen Pumpenkopf (50), einen innerhalb des Pumpenkopfes (50) sich durch eine Plungerdichtung (36) auf und ab bewegenden Plunger (49) und eine durch die Plungerdichtung (36) vom Pumpenkopf (50) getrennte geteilte Kammer (34), und
die gegeneinander gerichteten Flächen des Plungers (49) und die Plungerdichtung (36) durch Zuführen einer Reinigungsflüssigkeit in der geteilten Kammer (34) ausgewaschen werden.

3. Analysator zur Messung von glykolisiertem Hämoglobin nach Anspruch 2, wobei die Flüssigkeitszuführpumpe (11, 40) durch Zuführen der Reinigungsflüssigkeit mit der Spritzenpumpe (18, 37) durch das Flussdurchgang-Umschaltventil (17, 38) ausgewaschen wird.

4. Analysator zur Messung von glykolisiertem Hämoglobin nach Anspruch 1, ferner aufweisend:
eine Probenbehälter-Identifikationsvorrichtung zum Identifizieren von in einem Gestell (41) eingesetzten Behältern (43, 48), wobei die Probenbehälter-Identifikationsvorrichtung Mikroschalter (44, 45) vom Aktuatortyp aufweist, deren Anzahl gleich groß wie oder größer als die Anzahl Arten von zu identifizierenden Behältern (43, 48) ist,
wobei die Arten der Behälter (43, 48) durch die Ein- und Aus-Zustände der Mikroschalter (44, 45) vom Aktuatortyp identifiziert werden.

5. Analysator zur Messung von glykolisiertem Hämoglobin nach Anspruch 4, wobei die Probenbehälter-Identifikationsvorrichtung die Arten von Behältern (43, 48) während der intermittierenden Bewegung der im Gestell (41) eingesetzten Behälter (43, 48) identifiziert.

6. Analysator zur Messung von glykolisiertem Hämoglobin nach Anspruch 4, wobei die Probenbehälter-Identifikationsvorrichtung die Arten von Behältern auf Basis des Höhenunterschieds der im Gestell (41) eingesetzten Behälter (43, 48) identifiziert.

7. Analysator zur Messung von glykolisiertem Hämoglobin nach Anspruch 4, wobei die Arten von zu identifizierenden Behältern ein Blutentnahmeröhrchen umfassen

## Revendications

1. Analyseur d'hémoglobine glyquée utilisant une chromatographie en phase liquide, comprenant :
une partie d'échantillonnage (17, 18, 37, 38) pour une dilution automatique d'un échantillon de sang afin de créer un échantillon dilué pour l'analyse,
une soupape d'injection d'échantillon (12) pour alimenter en ledit échantillon dilué à analyser une colonne de séparation ;
une pompe d'alimentation en liquide (11, 40) pour l'alimentation en éluant ;
une colonne de séparation (14) pour séparer un composant d'hémoglobine ; et
un détecteur (15) pour détecter le composant séparé ;
**caractérisé en ce que** :
ladite partie d'échantillonnage (17, 18, 37, 38) comprend un mécanisme d'inversion de passage d'écoulement comprenant une soupape d'inversion de passage d'écoulement du type rotatif (17, 38), et une pompe seringue (18, 37),
dans lequel
ladite soupape d'inversion de passage d'écoulement est un type de soupape non-électromagnétique,
ledit mécanisme d'inversion de passage d'écoulement est construit de sorte qu'un passage d'écoulement soit formé par une inversion de ladite soupape d'inversion de passage d'écoulement,
ladite soupape d'inversion de passage d'écoulement (17, 38) a au moins trois orifices (1, 2, 3, 4, 5, 6, 7), et ladite soupape d'inversion de passage d'écoulement (17, 38) est inversée pour relier deux orifices (1, 2, 3, 4, 5, 6, 7) pour former ainsi le passage d'écoulement,
ladite pompe seringue (18, 37) est reliée à l'orifice central (7) de ladite soupape d'inversion de passage d'écoulement (17, 38),
une partie de solution d'hémolyse et de lavage (16) est reliée à un autre orifice (1, 2, 3, 4, 5, 6) de ladite soupape (17, 38) pour aspirer la solution d'hémolyse et de lavage (16) dans ladite pompe seringue (18, 37), et
ladite soupape d'injection d'échantillon (12) est reliée à un autre orifice (1, 2, 3, 4, 5, 6) de ladite soupape (17, 38)
(i) pour décharger ledit échantillon de sang aspiré dans une aiguille d'échantillonnage (20) à un bloc de dilution (22) pour le mélange et la dilution,
(ii) pour introduire un échantillon dilué à la soupape d'injection d'échantillon (12) et
(iii) pour nettoyer l'intérieur de l'aiguille d'échantillonnage (20).

2. Analyseur d'hémoglobine glyquée selon la revendication 1, dans lequel ladite pompe d'alimentation en liquide (11, 40) est une pompe à piston comportant une tête de pompe (50), un piston (49) effectuant un mouvement de va-et-vient à l'intérieur de ladite tête de pompe (50) à travers un joint d'étanchéité de piston (36) et une chambre cloisonnée (34) séparée de ladite tête de pompe (50) par ledit joint d'étanchéité de piston (36), et
les surfaces opposées dudit piston (49) et dudit joint d'étanchéité de piston (36) sont lavées en alimentant en liquide de nettoyage ladite chambre cloisonnée (34).

3. Analyseur d'hémoglobine glyquée selon la revendication 2, dans lequel ladite pompe d'alimentation en liquide (11, 40) est lavée par alimentation en liquide de nettoyage par ladite pompe seringue (18, 37) à travers la soupape d'inversion de passage d'écoulement (17, 38).

4. Analyseur d'hémoglobine glyquée selon la revendication 1, comprenant en outre :
un dispositif d'identification de récipient d'échantillon pour identifier des récipients (43, 48) fixés dans un support (41), ledit dispositif d'identification de récipient d'échantillon comportant des micro-rupteurs de type actionneur (44, 45), dont le nombre est supérieur ou égal au nombre de types de récipients (43, 48) à identifier,
dans lequel les types desdits récipients (43, 48) sont identifiés par les états passant et bloqué desdits micro-rupteurs de type actionneur (44, 45).

5. Analyseur d'hémoglobine glyquée selon la revendication 4, dans lequel ledit dispositif d'identification de récipient d'échantillon identifie les types desdits récipients (43, 48) pendant le mouvement intermittent desdits récipients (43, 48) fixés dans le support (41).

6. Analyseur d'hémoglobine glyquée selon la revendication 4, dans lequel ledit dispositif d'identification de récipient d'échantillon identifie les types desdits récipients sur la base de la différence de hauteur desdits récipients (43, 48) fixés dans le support (41).

7. Analyseur d'hémoglobine glyquée selon la revendication 4, dans lequel les types desdits récipients à identifier comportent un tube de prélèvement sanguin.
